# Europäisches Patentamt

# European Patent Office

# Office européen des brevets

(19)

(11) Veröffentlichungsnummer : **0 290 566 B1**

(12) **EUROPÄISCHE PATENTSCHRIFT**

(45) Veröffentlichungstag der Patentschrift :
05.02.92 Patentblatt 92/06

(51) Int. Cl.⁵ : **A61F 9/00,** A61B 3/12

(21) Anmeldenummer : **87907939.0**

(22) Anmeldetag : **09.11.87**

(86) Internationale Anmeldenummer :
**PCT/DE87/00589**

(87) Internationale Veröffentlichungsnummer :
**WO 88/03396 19.05.88 Gazette 88/11**

(54) **VORRICHTUNG ZUR ERZEUGUNG VON BILDERN EINES OBJEKTS UND INSBESONDERE ZUR BEOBACHTUNG DER HINTEREN AUGENABSCHNITTE.**

(30) Priorität : **08.11.86 DE 3638226**

(43) Veröffentlichungstag der Anmeldung :
**17.11.88 Patentblatt 88/46**

(45) Bekanntmachung des Hinweises auf die
Patenterteilung :
**05.02.92 Patentblatt 92/06**

(84) Benannte Vertragsstaaten :
**AT DE FR GB IT**

(56) Entgegenhaltungen :
**EP-A- 0 145 563**
**WO-A-87/05204**
**DE-A- 3 306 981**
**US-A- 4 213 678**
**IEEE Engineering in Medicine and Biology**
**Magazine, Band 4, Nr. 4, Dezember 1985, IEEE,**
**(New York, US), R.H. Weeb: "Manipulating**
**laser light for ophtalmology", Seiten 12-16**

(73) Patentinhaber : **G. RODENSTOCK**
**INSTRUMENTE GMBH**
**Drachenseestr. 10 - 12**
**W-8000 München 70 (DE)**

(72) Erfinder : **FEUERSTEIN, Manfred**
**Gottfried-Böhm-Ring 23**
**W-8000 München 70 (DE)**
Erfinder : **KLINGBEIL, Ulrich**
**Daglfingerstr. 108**
**W-8000 München 81 (DE)**
Erfinder : **KÜHL, Claus-Heinrich**
**Schieggstr. 20**
**W-8000 München 70 (DE)**
Erfinder : **PLESCH, Andreas**
**Schinkelstr. 1**
**W-8000 München 40 (DE)**

(74) Vertreter : **Münich, Wilhelm, Dr. et al**
**Kanzlei Münich, Steinmann, Schiller**
**Willibaldstrasse 36/38**
**W-8000 München 21 (DE)**

EP 0 290 566 B1

## Beschreibung

### Technisches Gebiet

Die Erfindung bezieht sich auf eine Vorrichtung zur Erzeugung von Bildern eines Objekts und insbesondere zur Beobachtung der hinteren Augenabschnitte gemäß dem Oberbegriff des Anspruchs 1.

### Stand der Technik

Vorrichtungen gemäß dem Oberbegriff des Anspruchs 1 sind allgemein bekannt und werden in den verschiedensten Gebieten zur Erzeugung von Bildern eingesetzt. Beispielsweise in der Medizintechnik sind eine Reihe von Laser-Scanning-Kameras, Laser-Scanning-Mikroskopen und Laser-Scanning-Ophthalmoskopen vorgeschlagen worden.

Vorrichtungen gemäß dem Oberbegriff des Anspruchs 1 haben sich insbesondere dann als vorteilhaft herausgestellt, wenn ein vergleichsweise großes Objekt durch eine kleine vor dem Objekt angeordnete Blende betrachtet werden muß: Beispielsweise bei der Beobachtung der hinteren Augenabschnitte besteht die Schwierigkeit, daß die Beleuchtung und die Beobachtung des Fundus durch die Augenpupille und die häufig optisch nicht klaren vorderen Augenmedien erfolgen muß, an denen Reflexe auftreten, und die Abbildungsfehler erzeugen. Ähnliche Verhältnisse sind aber auch in anderen medizinischen oder technischen Einsatzfällen gegeben.

In der Vergangenheit sind deshalb zur Beobachtung der hinteren Augenabschnitte meist Funduskameras verwendet worden, bei denen zur Unterdrückung des sog. Corneareflexes die Eintritts- und die Austrittspupille nach "GULLSTRAND" separiert sind: Der für die Beleuchtung verwendete Teil der Augenpupille umgibt ringförmig den für die Beobachtung verwendeten Teil.

Trotzdem können bei diesen bekannten Funduskameras Reflexe nicht vollständig unterdrückt werden. Zudem ist die erreichbare Auflösung von ca 15 μm häufig unzureichend.

Deshalb ist bereits mehrfach vorgeschlagen worden, zur Beobachtung des Augenhintergrunds Vorrichtungen gemäß dem Oberbegriff des Anspruchs 1 zu verwenden, die den Augenhintergrund nicht großflächig auszuleuchten, sondern mit auf einen möglichst kleinen Fleck fokussierten Beleuchtungslicht abzutasten und das reflektierte Licht in Zuordnung zur Abtastsequenz zu erfassen. Hierzu wird beispielsweise auf "The foundations of Opthalmology", Bd. VII, S.307/308, Jg. 1962, die US-A-4 213 678, die EP-A-0 145 563 sowie die japanischen Patentveröffentlichungen 61-5730 und 50-138822 verwiesen.

Die aus den genannten Fundstellen bekannten Vorrichtungen unterscheiden sich u.a. in der Pupillenseparation: so werden in der japanischen Patentveröffentlichung 61-5730 eine "GULLSTRAND-Pupille", in der US-A-4 213 678 eine invertierte "GULLSTRAND-Pupille" und in der jaganischen Patentveröffentlichung 50-138822 nebeneinanderliegende Pupillen für das Beleuchtungs- und das Beobachtungslicht vorgeschlagen.

Bei der in der EP-A-0 145 563 beschriebenen Vorrichtung zur Beobachtung der hinteren Augenabschnitte sind sowohl der Beleuchtungs- als auch der Beobachtungslichtstrahl über die Abtasteinrichtung geführt. Ein derartiges "Double-Scanning-System" hat den Vorteil, daß der reflektierte Lichtstrahl mit einem ortsfesten Detektor mit vergleichsweise kleiner Fläche nachgewiesen werden kann.

Den genannten Vorrichtungen zur Beobachtung der hinteren Augenabschnitte mit "scannender Beleuchtung" ist gemeinsam, daß die Auflösung des erhaltenen Bildes durch die Größe des "Fokusfleckes" (ca 8-12μm) auf dem Augenhintergrund bestimmt ist, und daß das reflektierte Licht mit einem einzigen Detektor mit einer mehr oder weniger großen Bildfeldblende zum Aufbau des Bildes der hinteren Augenabschnitte erfaßt wird.

Eine weitergehende Analyse des rückgestreuten Lichts ist bislang nicht in Betracht gezogen worden.

### Darstellung der Erfindung

Die Erfindung geht von der Erkenntnis aus, daß es gerade bei Vorrichtungen zur Erzeugung von Bildern gemäß dem Oberbegriff des Anspruchs 1 durch besondere Arten der Beleuchtung und/oder der Analyse des rückgestreuten Lichts nach den verschiedensten Kriterien möglich ist, wesentlich weitergehende Informationen über das abzubildende Objekt, beispielsweise den Augenhintergrund zu erhalten als dies mit irgendeiner anderen der bekannten Vorrichtungen zur Bilderzeugung möglich ist.

Der Erfindung liegt deshalb die Aufgabe zu Grunde, eine Vorrichtung zur Erzeugung von Bildern eines Objekts gemäß dem Oberbegriff des Anspruchs 1 derart weiterzubilden, daß durch besondere Arten der Beleuchtung und/oder der Analyse des rückgestreuten Lichts eine über die reine Bilderzeugung hinausgehende Analyse des abzubildenden Objekts möglich wird.

Eine erfindungsgemäße Lösung dieser Aufgabe ist mit ihren Weiterbildungen in den Patentansprüchen gekennzeichnet.

Beispielsweise ermöglicht die im Anspruch 1 gekennzeichnete Vorrichtung eine räumliche Analyse und-/oder eine Analyse des Polarisationszustandes des rückgestreuten Lichts. Hierzu weist die erfindungsgemäße Vorrichtung eine Detektoreinrichtung mit mehreren Einzeldetektoren auf, die zur Erfassung des von unterschiedlichen Ebenen reflektierten Lichts und/oder zur Erfassung der flächenhaften Intensitätsverteilung des Lichts in einer Ebene und/oder zur Erfassung des Polarisationszustandes des Lichts konjugiert zu unterschiedlichen Ebenen bzw. zu unterschiedlichen Bereichen einer Ebene angeordnet bzw. denen entsprechende Blenden oder Polarisationsfilter vorgeschaltet sind.

Diese Ausbildung der erfindungsgemäßen Vorrichtung erlaubt damit eine Messung der räumlichen Intensitätsverteilung und/oder des Polarisationszustandes des an dem abzubildenden Objekt, beispielsweise den hinteren Augenabschnitten reflektierten Lichts. Darüberhinaus ist durch die Anordnung von Detektoren in zu verschiedenen Ebenen des abzubildenden Objekts konjugierten Ebenen eine Tiefenanalyse des Objekts möglich.

Ausdrücklich soll an dieser Stelle klargestellt werden, daß es bei der vorliegenden Erfindung, wenn davon die Rede ist, daß Detektoren eine bestimmte Form (Umriß) haben oder an einer bestimmter Stelle angeordnet sind, nicht erforderlich ist, daß die Detektoren tatsächlich entsprechend ausgebildet sind. Es genügt vielmehr, wenn gemäß Anspruch 34 an der entsprechenden Stelle bildfeldbestimmende Blenden angeordnet sind, die mit den Detektoren über lichtleitende Mittel, beispielsweise Relaisoptiken oder Lichtleiter (Anspruch 35) verbunden sind. Diese Anordnung bildfeldbestimmender Blenden anstelle von Detektoren ist eine besondere Eigenschaft des zum Bildaufbau verwendeten "Scan-Verfahrens", bei dem keine eigentliche Abbildung erfolgt, sondern das zu jedem Zeitpunkt in den gesamten Raumwinkel bzw. den erfaßbaren Raumwinkel reflektierte bzw. gestreute Licht erfaßt und zeitsequentiell zum Bildaufbau ausgewertet wird.

Bei der im Anspruch 2 gekennzeichneten Lösung der erfindungsgemäß gestellten Aufgabe wird Licht mehrerer Wellenlängen, vorzugsweise das Licht mehrerer Laser gleichzeitig auf die gleiche Stelle des abzubildenden Objekts projiziert. Hierdurch können die verschiedensten Wirkungen erzielt werden:

Beispielsweise ist es bei einem Laser-Scanning-Ophthalmoskop möglich, eine Weißlichtbeleuchtung zu simulieren und mit einer "Echtfarb-Darstellung" auf einem Monitor dem Augenarzt subjektiv das "gewohnte" Bild des Augenhintergrundes zu liefern.

Darüberhinaus ist es bei geeigneter Wahl der Wellenlängen möglich, beispielsweise die Blutsauerstoffsättigung zu bestimmen, die Störungen der lokalen Durchblutung und des gesamten Kreislaufs, avaskuläre Zonen etc. zeigt. Ferner ist eine Tumoranalyse, eine Sehpigmentanalyse etc möglich.

Weiterhin ist es bei gleichzeitiger Verwendung eines $Ar^+$- Lasers oder eines HeNe-Lasers sowie eines Lasers mit einer abweichenden Wellenlänge möglich, gleichzeitig ein Angiofluoreszenz- und ein "normales" Bild des Fundus zu erhalten.

Die gleichzeitige Verwendung von Licht mehrerer Wellenlängen ermöglicht darüberhinaus noch folgende interessante Möglichkeit:

Die Schärfentiefe des Beleuchtungslichts hängt von der Eintrittspupille, d.h. von der Größe und der Form der Pupille für das Beleuchtungslicht ab. Beispielsweise bei einer als invertierte Gullstrand-Pupille ausgebildeten Eintrittspupille, wie sie in der US-A- 4 213 678 vorgeschlagen worden ist, erhält man aufgrund des kleinen Randwinkels des Beleuchtungslichts eine große Schärfentiefe. Andererseits erhält man bei Verwendung einer normalen Gullstrand-Pupille oder der in der EP-A-0 145 563 verwendeten Pupille aufgrund des großen von den Randstrahlen eingeschlossenen Winkels eine kleine Schärfentiefe.

Normalerweise wird die Eintrittspupille entsprechend den jeweiligen Vorgaben gewählt, wobei die invertierte Gullstrand-Pupille die beste Auflösung liefert, da bei ihr die optisch schlechteren Randbereiche des Auges nicht für die Beleuchtung verwendet werden und somit das Beleuchtungslicht auf den kleinsten Fleckdurchmesser fokussierbar ist.

Bei Verwendung von mehreren Lichtquellen, die Licht unterschiedlicher Wellenlänge liefern, können nun gemäß Anspruch 3 unterschiedliche Eintrittspupillen verwendet werden, wobei es gemäß Anspruch 4 besonders bevorzugt ist, wenn für Licht der einen Wellenlänge eine Eintrittspupille verwendet wird, die eine große Schärfentiefe liefert, sowie für Licht einer anderen Wellenlänge eine Eintrittspupille, die eine kleine Schärfentiefe liefert. Damit ist es gleichzeitig möglich, ein Übersichtsbild mit hoher Auflösung und großer Schärfentiefe und ein zweites Bild zu erhalten, das "tiefenselektiv" ist. Dabei kann die Wahl der unterschiedlichen Ein- und gegebenenfalls Austrittspupillen beispielsweise durch eine geeignete wellenlängenselektive Beschichtung des sog. optischen Einkoppelelements (Teilerspiegels), d.h. des den Beleuchtungs- und Beobachtungsstrahlengang trennenden Spiegels erfolgen. In diesem Falle erhält man für die verschiedenen Wellenlängen komplementäre Pupillen. Natürlich ist aber auch eine andere Aufteilung der Pupillen durch geeignete Maßnahmen, beispielsweise die Verwendung mehrerer Teilerspiegel möglich, so daß auch andere Pupillenteilungen als komplemtäre Pupillen möglich sind.

Die Signale der verschiedenen Detektoren, also beispielsweise ein Angiographiebild und das "normale Bild" können auf einem Monitor überlagert oder auf mehreren Monitoren dargestellt werden (Anspruch 5).

In jedem Falle kann die Darstellung beider oder mehrerer Bilder in Echtzeit (Anspruch 6) oder nach Speicherung (Anspruch 7) verknüpft werden. Unter "Verknüpfen" werden dabei die in der Bildverarbeitung (Anspruch 32) bekannten Operationen verstanden, beispielsweise erhält man sehr aussagekräftige Bilder durch eine "Echtzeit-Überlagerung" eines Angiographie-Bildes und des normalen Bildes. Die einzelnen direkt oder nach Bildverarbeitung erhaltenen Bilder können natürlich gleichzeitig auf mehreren Beobachtungseinrichtungen, beispielsweise Monitoren dargestellt und/oder aufgezeichnet werden.

Dabei ist es gemäß Anspruch 8 besonders vorteilhaft, die erfindungsgemäße Vorrichtung so auszubilden, daß sowohl das Beleuchtungslicht als auch das reflektierte Licht über die Abtasteinrichtung geührt wird, da bei einer derartigen Vorrichtung in einfacher Weise nach der Abtasteinrichtung ein Nachweis-Lichtsignal erhalten wird, das seine Lage im Raum nicht ändert.

Wie bereits ausgeführt worden ist, kann erfindungsgemäß die räumliche Verteilung des reflektierten Lichts erfaßt und ausgewertet werden: Hierzu ist es insbesondere möglich, Detektoren bzw. bildfeldbestimmende Blenden in Ebenen anzuordnen, die nicht zu der eigentlichen Objektebene konjugiert sind.

Beispielsweise ist gemäß Anspruch 9 in einer zur Pupille des Auges konjugierten Ebene eine Detektoranordnung bzw. eine bildfeldbestimmende Blende vorgesehen, die die Intensitätsverteilung des reflektierten Lichts in dieser Ebene erfaßt. Die Einzeldetektoren bzw. die Blendenelemente haben dabei vorzugsweise die Form von Kreissektoren (Anspruch 11), so daß der Schwerpunkt des reflektierten bzw. rückgestreuten Lichts, Richtungsasymmetrien usw. ermittelt werden können, wodurch beispielsweise Rückschlüsse auf Oberflächenstrukturen möglich sind (Anspruch 10).

Bei der im Anspruch 12 gekennzeichneten Weiterbildung ist in einer zum abzubildenden Objekt, also beispielsweise zum Augenhintergrund konjugierten Ebene eine Detektoranordnung bzw. eine Blendenanordnung vorgesehen, die die Intensitätsverteilung des reflektierten Lichts in dieser Ebene erfaßt.

Hierdurch ist es beispielsweise möglich, den Anteil der Querstreuung in der Retina durch eine Analyse der Intensitätsverteilung in einer zur Retina konjugierten Bildebene zu ermitteln und dadurch Aufschluß über Netzhaut-Strukturen zu erhalten (Anspruch 13).

Neben der räumlichen Analyse des reflektierten Lichts ist durch die im Anspruch 14 gekennzeichnete Ausgestaltung auch eine Analyse des Polarisiationszustandes des reflektierten Lichts möglich, so daß sich gegenüber den bekannten Vorrichtungen eine verbesserte Darstellung der doppelbrechenden Nervenfaserschicht der Retina ergibt.

Die im Anspruch 15 angegebene Weiterbildung erlaubt darüberhinaus die Ermittlung der Stokes-Parameter zur Beschreibung der Polarisationscharakteristik, durch die lokale Defekte ermittelt und anisotrope, z.B. gerichtete Strukturen der Retina, wie etwa die Nervenfaserschicht hervorgehoben werden können. Im übrigen wird bezüglich der Definition der Stokes-Parameter auf den Artikel "Polarization imaging" in APPLIED OPTICS, Vol. 20, S.1537 folgende verwiesen.

In den Ansprüchen 16 bis 19 sind vorteilhafte Weiterbildungen angegeben, die eine optische Strukturanalyse bzw. eine optische Bildvorverarbeitung des reflektierten Lichts ermöglichen. Die erfindungsgemäß erstmals bei einer Vorrichtung gemäß dem Oberbegriff des Anspruchs 1 vorgesehene optische Bildvorverarbeitung arbeitet wesentlich schneller als verfügbare elektronische Bildverarbeitungssysteme und ermöglicht damit selbst bei komplexen Filterungen eine Hervorhebung spezieller Objektstrukturen in Echtzeit !

Je nach Position des analysierenden Filters bzw. der Blende kann man Polarisationszustände isolieren, Richtungsanisotropien selektieren, Aberrationen kompensieren etc. Besonders vorteilhaft ist es, wenn variable, beispielsweise von einem Rechner angesteuerte Filter und/oder Blenden (Anspruch 17), austauschbare und-/oder drehbare (Anspruch 19) sowie Blenden mit gradueller Transmission (Apodisierung) vorgesehen sind. Diese Blenden sind in der Regel keine einfachen Lochblenden, sondern je nach Einsatzfall geformte Anordnungen, beispielsweise Schlitze, Ringe, Quadrantenkombinationen oder ein Muster von einzeln angesteuerten Punkten. Ferner können die Blenden teilweise verspiegelte Glasglatten, durchbrochene Spiegel, wellenlängenselektiv bedampfte Spiegel oder teildurchlässige Spiegel sein. Auch ist es möglich, durch eine entsprechende Gestaltung des Teilerspiegels unterschiedlich optisch wirksame Zonen auszubilden !

Im Anspruch 18 ist ein einfach aufgebauter Lichtmodulator, nämlich ein Flüssigkristallelement gekennzeichnet, das bereichsselektiv lichtdurchlässig bzw. lichtundurchlässig geschaltet werden kann.

Im Anspruch 20 ist eine weitere bevorzugte Lösung der erfindungsgemäß gestellten Aufgabe und insbesondere eine Ausbilung einer Vorrichtung zur Beobachtung der hinteren Augenabschnitte gekennzeichnet, die sich dadurch auszeichnet, daß zusätzlich Marken auf das zu beobachtende Objekt, also beispielsweise den Augenhintergrund projiziert werden können. Diese Marken können beispielsweise zur Markierung von zu behandelnden (koagulierenden) oder zu untersuchenden Gebieten dienen und mittels Bildverarbeitung in einem vorher aufgenommenen Bild erzeugt werden. Hierzu wird ausdrücklich auf die ältere Patentanmeldung DE-A-6 07 721.6 verwiesen, in der die Verwendug von Bildverarbeitung zur Behandlungsplanung und zur Erzeugung von Markierungen ausführlich erläutert ist.

Diese Marken können beispielsweise durch "Hellschalten" des Beleuchtungslichtstrahls an der entsprechenden Stelle erzeugt werden (Anspruch 21). Das Hellschalten ist besonders dann bevorzugt, wenn die Marken zur Orientierung und/oder Kennzeichnung eines bestimmten Bereichs dienen sollen, da dann in jedem Falle die Ortsbeziehung zwischen "Scan-Strahl" und Marke erhalten bleibt.

Es ist jedoch auch möglich, zur Projektion der Marken eine eigene Lichtquelle und insbesondere eine eigene Positionierungseinheit (Anspruch 22) zu verwenden, da dann eine weitestgehende Unabhängigkeit der einzelnen Systeme gewährleistet ist. Diese Ausbildung ist besonders dann empfehlenswert, wenn der zusätzliche Strahl zu Bearbeitungszwecken, beispielsweise Koagulationszwecken dienen soll.

Dieses Positionierungssystem kann beispielsweise ein akustooptischer Deflektor oder eine Taumeloptik nach Patentanmeldung DE-A-35 32 464.3, da dieser einfach und stabil zu handhaben ist (Anspruch 23).

Als Abtasteinrichtung wird dagegen erfindungsgemäß bevorzugt ein x/y-Scanner mit einer Polygon-Spiegeltrommel und einem Galvanometerspiegel verwendet, da ein derartiges System wellenlängenunabhängig arbeitet, was insbesondere bei der erfindungsgemäß vorgesehenen gleichzeitigen Verwendung von Licht mehrerer Wellenlängen von Vorteil ist (Anspruch 25).

Neben den vorstehend erläuterten Anwendungsmöglichkeiten für die zusätzlich eingespiegelten Markierungen ist eine weitere Anwendungsmöglichkeit die im Anspruch 24 genannte Fundusperimetrie. Die Markierungen dienen dabei als sog. Stimuli, die der Patient erkennt bzw. bei Gesichtsfeldausfällen nicht erkennt. Dabei ist es besonders vorteilhaft, wenn als Beobachtungslichtquelle ein Infrarotlaser verwendet wird, da dann das Beobachtungslicht nicht die Wahrnehmung der Stimuli beeinträchtigt. Durch diese erfindungsgemäße Weiterbildung einer Vorrichtung zur Beobachtung der hinteren Augenabschnitte erhält man ein Fundusperimeter, das Mikroperimetrie unter Sichtkontrolle ermöglicht und das darüberhinaus auch Sehschulung zur Behebung von Sehoder Fixationsschwächen erlaubt.

Bei der Mikroperimetrie, aber nicht nur bei dieser Anwendung der erfindungsgemäßen Vorrichtung, ist es weiterhin von Vorteil, wenn eine zusätzliche Umfeldbeleuchtung vorgesehen wird, die beispielsweise über einen teildurchlässigen Spiegel eingekoppelt wird. Diese Umfeldbeleuchtung erlaubt beispielsweise Perimetrie auf einem "bestimmten Helligkeitslevel", hat aber natürlich auch noch weitere Vorteile.

Bei der Fundusperimetrie können über eine Steuereinheit besondere Suchalgorithmen, Skotomerkennung, eine variable Meßpunktdichte, Fundus-Tracking zur automatischen Lageerkennung der projizierten Marken auf dem Fundus realisiert werden. Ferner können eine Positiv- und Negativperimetrie, Farbdifferentialuntersuchungen etc durchgeführt werden.

Bei einer weiteren erfindungsgemäßen Ausbildung einer Vorrichtung, die insbesondere zur Beobachtung der hinteren Augenabschnitte geeignet ist, sind gemäß Anspruch 30 ein oder mehrere Detektoren vorgesehen, denen Blenden derart vorgeschaltet sind, daß sich eine Dunkelfeldbeleuchtung der Detektoren ergibt, so daß nur die mehrfachgestreuten Komponenten im Objekt- bzw. Fundusbild aufgenommen werden. Hierdurch erhält man eine kontrastverstärkte Darstellung bestimmter Strukturen, z.B. dem Papillengewebe ermöglicht. Dies ist für die automatische Papillenrandbestimmung im Rahmen der Glaukomdiagnostik von hohem Interesse.

Durch das erfindungsgemäße Konzept, mehrere Detektoren vorzusehen, ist es selbstverständlich möglich, "Dunkelfeld"- und "Hellfeld"-Bilder gleichzeitig aufzunehmen.

Ferner ist es erfindungsgemäß von Vorteil, wenn gemäß Anspruch 31 in das aufgenommene und dargestellte Bild früher aufgenommene Bilder und/oder Markierungen deckungsgleich eingespiegelt werden, so daß der Bedienungsperson beispielsweise ein Vergleich mit auf andere Weise aufgenommenen Bildern, beispielsweise Angiographien oder eine Kontrolle einer automatischen Laserstrahlpositionierung möglich wird. Hierzu wird ebenfalls auf die ältere Patentanmeldung DE-A-36 07 721.6 verwiesen. Das "Einspiegeln kann optisch, bevorzugt aber elektronisch in die Beobachtungseinrichtung, beispielsweise einen Monitor erfolgen.

Die erfindungsgemäße Vorrichtung eignet sich nicht nur als Bilderzeugungs- bzw. Diagnose-, sondern auch als Bearbeitungs- bzw. Therapiegerät und kann mit den verschiedensten Instrumenten kombiniert werden, beispielsweise Bearbeitungs- bzw. Behandlungslasern unterschiedlicher Wellenlänge.

Besonders vorteilhaft ist es jedoch, wenn zusätzlich der Strahl eines Koagulationslaser, also beispielsweise eines Ar⁺-Lasers oder eines Farbstofflasers bevorzugt zwischen Abtasteinrichtung und Auge eingespiegelt wird (Anspruch 27). Natürlich ist es aber auch möglich, zum Koagulieren die Leistung eine Beobachtungslasers "kurzfristig" zu erhöhen, wie dies in der US-A- 4 213 678 beschrieben ist. Die Mitverwendung der "Scan-Einrichtung" ermöglicht in diesem Falle insbesondere die Behandlung größerer Gebiete bzw. mehrerer Gebiete In einem Schritt.

Eine Vorrichtung zur Beobachtung der hinteren Augenabschnitte mit abtastender Beleuchtung ist wegen der reflexfreien und hochauflösenden Bilddarstellung besonders als Bildgeber für eine sog. Eye-Tracking-Einheit prädestiniert. Bezüglich des prinzipiellen Eye-Tracking-Konzepts -Nachführung der Beobachtungs- und-/oder Behandlungseinheit, Abschalten des Lasers bei Augenbewegungen usw. - wird wiederum auf die ältere Patentanmeldung DE-A-36 07 721.6 verwiesen, deren Inhalt im übrigen ausdrücklich als Offenbarung für diese Anmeldung beansprucht wird.

Weiterhin ist eine Vorrichtung zur Beobachtung der hinteren Augenabschnitte mit abtastender Beleuchtung wegen der reflexfreien und hochauflösenden Bilddarstellung besonders als Bildgeber für eine Behandlungsplanung geeignet wie sie ebenfalls bereits in der Patentanmeldung DE-A-36 07 721.6 beschrieben ist. Dies gilt insbesondere bei einer Ausgestaltung gemäß Anspruch 33.

Besonders vorteilhaft ist es jedoch, eine Vorrichtung gemäß mit "Double-Scanning" zu verwenden, die gemäß Anspruch 33 weitergebildet ist. Dabei wird im Gegensatz zu der aus der EP-A-0 145 563 bekannten Vorrichtung eine Bildfeldblende mit einem Durchmesser von 80 bis 150 µm verwendet, also wesentlich größer als der Punktbilddurchmesser auf dem Augenhintergrund, der typischerweise zwischen 8 und 12 µm, höchstens jedoch ca 20 µm beträgt. Durch diese Wahl der Bildfeldblende, die von der in der EP-A-0 145 563 als Erfindung beanspruchten Wahl abweicht, wird das erfindungsgemäße Konzept, mehrere Einzeldetektoren zu verwenden, besonders unterstützt. Die Pupillenseparation in der Pupillenebene kann dabei In aus den einzelnen Fundstellen bekannter Weise erfolgen.

## Kurze Beschreibung der Zeichnung

Die Erfindung wird nachstehend anhand von Ausführungsbeispielen unter Bezugnahme auf die Zeichnung näher beschrieben, in der zeigen:

Fig. 1 den allgemeinen Strahlengang einer erfindungsgemäßen Vorrichtung,
Fig. 2 den Strahlengang "vor" der Abtasteinrichtung,
Fig. 3 eine mögliche Detektoranordnung in einer zum Objekt konjugierten Ebene,
Fig. 4a - c mögliche Detektoranordnungen in einer zur Pupille konjugierten Ebene, und
Fig. 5 mögliche Pupillen für das Beleuchtungslicht und das reflektierte Licht.

## Darstellung von Ausführungsbeispielen

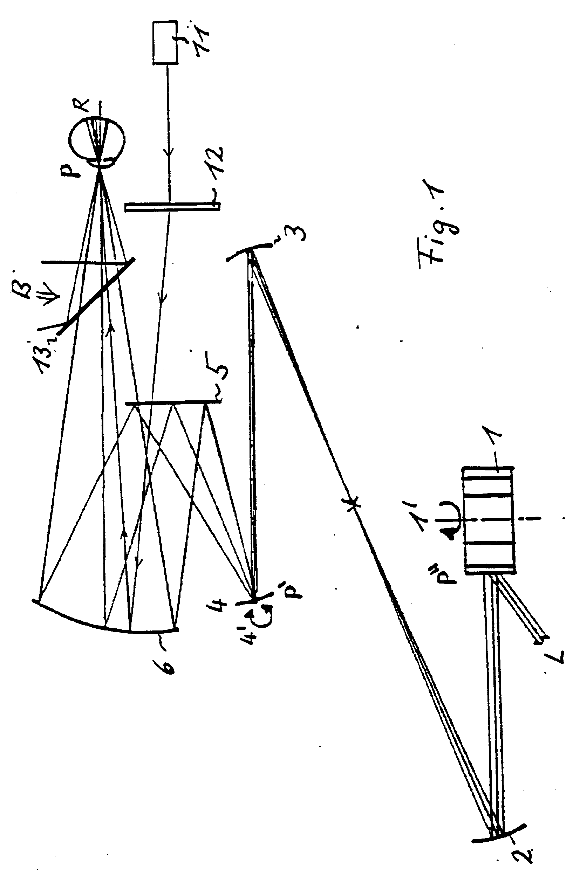

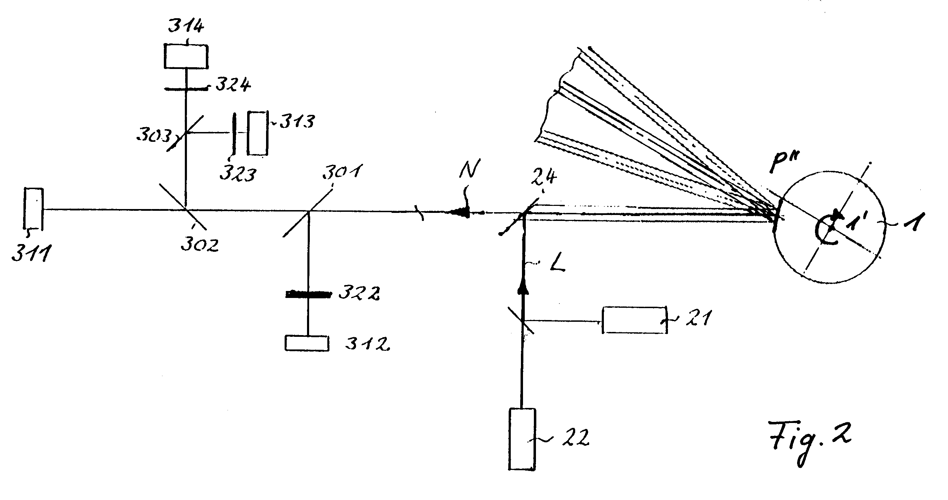

Fig. 1 zeigt den allgemeinen Strahlengang einer erfindungsgemäßen Vorrichtung, die ohne Beschränkung des allgemeinen Erfindungsgedankens als Laser-Scanning-Ophthalmoskop verwendet wird. Das Licht L einer in Fig. 2 näher dargestellten Beleuchtungs-Einrichtung trifft auf einen Polygonspiegel 1 einer Abtasteinrichtung auf. Der Polygonspiegel 1 lenkt entsprechend seiner Drehung in Richtung eines Pfeils 1' das Licht L in Horizontalrichtung ab. Ein Konkavspiegel 2 und ein weiterer Konkavspiegel 3 bilden das in Horizontalrichtung abgelenkte Lichtbündel auf einen Galvanometerspiegel 4 ab, der in Richtung eines Pfeils 4' schwingt und das Lichtbündel zusätzlich in Vertikalrichtung ablenkt. Das in Horizontal- (x) und in Vertikalrichtung (y) abgelenkte Lichtbündel wird von einem Planspiegel 5 umgelenkt und von einem Konkavspiegel 6 auf den Fundus (Retina) R eines Auges 7 derart fokussiert, daß der in x-und y-Richtung abtastende Lichtstrahl einen "Knotenpunkt" in der Pupillenebene P des Auges 7 hat. Die Brechkräfte der Elemente 2, 3, und 6 sowie die optischen Wege zwischen die Elementen sind so bemessen, daß die Ebenen P' bzw. P" der Spiegel 4 bzw. 1 konjugierte Ebenen zu der Pupillenebene P des Auges 7 sind.

Das am Augenhintergrund R reflektierte bzw. gestreute Licht N wird auf dem umgekehrten Weg über die Spiegel 6, 5, 4, 3 und 2 zum Polygonspiegel 1 zurückgeleitet und kann - wie in Fig. 2 näher dargestellt ist - "hinter" der Abtasteinrichtung mit einer ortsfesten Detektoranordnung nachgewiesen werden.

Ferner sind eine unabhängige Lichtquelle 11, die bevorzugt ein Laser ist, sowie eine von der Ablenkeinrichtung 1,4 unabhängig arbeitende Ablenkeinheit 12 vorgesehen, die den Strahl M der Lichtquelle 11 ablenkt und ein Positionieren des Strahls M auf der Retina erlaubt. Hierzu ist bei dem gezeigten Ausführungsbeispiel der Spiegel 5 als teildurchlässiger Spiegel ausgebildet.

Die Lichtquelle 11 kann beispielsweise ein Koagulationslaser, also z.B. ein $Ar^+$-Laser, ein Bildmarkengeber oder die Lichtquelle sein, die die Durchführung einer Mikro-Fundusperimetrie erlaubt.

Die Ablenkeinheit 12 kann natürlich jede beliebige Einheit sein, die das Positionieren eines Lichtstrahls auf einer Objektfläche ermöglicht. Dei Ablenkeinheit 12 kann ein akusto-optischer Deflektor oder eine Taumeleinheit sein.

Darüberhinaus ist im Strahlengang ein weiterer teildurchlässiger Spiegel 13 angeordnet, der das großflächige Einspiegeln des Lichts B einer weiteren, nicht dargestellten Beleuchtungsquelle erlaubt. Das Licht B dient zum großflächigen Beleuchten des Augenhintergrunds R und kann Insbesondere bei der Mikroperimetrie dazu dienen, einen bestimmten Helligkeitslevel "einzustellen", auf dem dann die mit dem Laser 11 eingespiegelten Marken erkannt werden müssen.

Fig. 2 zeigt die Ausbildung der Beleuchtungs-Einrichtung und der Detektoreinrichtung, die im Lichtweg "vor" bzw. "hinter" dem Polygonspiegel 1 der Abtasteinrichtung angeordnet sind.

Bei dem gezeigten Ausführungsbeispiel weist die Beleuchtungseinrichtung zwei Laser 21 und 22 auf, die Licht unterschiedlicher Wellenlänge, beispielsweise im UV-und sichtbaren Bereich oder im sichtbaren und Infrarot-Bereich ausstrahlen.

Der Lichtweg der beiden Laser wird mittels eines teildurchlässigen oder wellenlängenselektiven Spiegels 23 vereinigt und mittels eines Teilerspiegels 24 in den gemeinsamen Lichtweg des Beleuchtungslichts L und des Nachweislichts N eingekoppelt.

Die Ausbildung des Teilerspiegels 24 bestimmt die Ausbildung der Eintrittspupille, d.h. des Teils der Augenpupille, den das Beleuchtungslicht L durchsetzt, und der Austrittspupille, d.h. des Teils der Augenpupille, den das an der Retina R reflektierte bzw. gestreute Licht N durchsetzt.

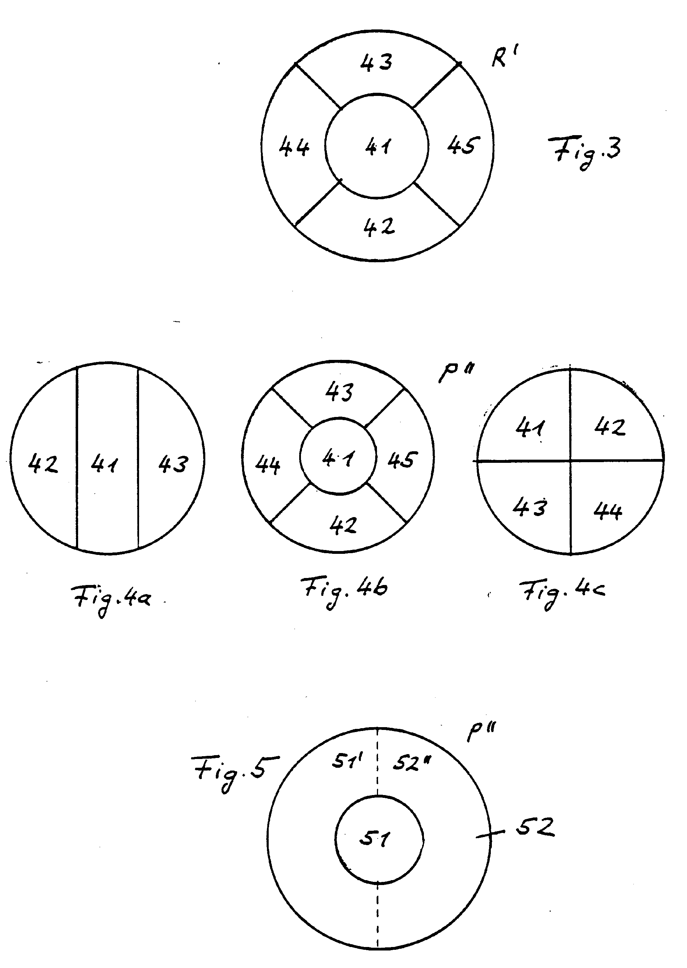

Fig. 5 zeigt eine mögliche Pupillenteilung. Durch eine wellenlängenselektive Beschichtung des Teilerspiegels 24 kann erreicht werden, daß das Licht eines beispielsweise im sichtbaren Bereich arbeitenden Lasers lediglich von dem die optische Achse umgebenden Bereich reflektiert wird, so daß die Eintrittspupille der Bereich 51 ist. Der den Bereich 51 umgebende Bereich 52 ist dann die Austrittspupille. Wählt man die Spiegelschichten des Spiegels 24 derart, daß die für sichtbares Licht reflektierende Schicht Licht beispielsweise im Infrarotbereich durchläßt und umgekehrt, so ist die Eintrittspupille für Licht im Infrarotbereich der Bereich 52 und der Bereich 51 die Austrittspupille.

Aufgrund der unterschiedlichen Winkel der Randstrahlen wird das durch den Bereich 51 hindurchtretende Licht auf der Retina R mit einer hohen Schärfentiefe fokussiert, während das durch den Bereich 52 einfallende Licht mit einer geringen Schärfentiefe fokussiert wird. Damit lassen sich mit zwei Lasern 21 und 22 gleichzeitig Bilder mit hoher Schärfentiefe und Bilder mit geringer Schärfentiefe im Bereich von 0,1mm und darunter aufnehmen, die eine Tiefenanalyse gestatten, während das gleichzeitig aufgenommene Bild mit hoher Schärfentiefe eine Überblicksdarstellung ermöglicht.

Ferner ist in Fig. 5 angedeutet, daß die Austrittspupille 52 in zwei Bereiche 52' und 52" aufgeteilt werden kann. Die Differenz der in den Bereichen 52' und 52" aufgenommenen Signale ermöglicht eine Aussage über die Richtungsasymmetrie der Querstreuung.

Weiterhin ist in Fig. 2 exemplarisch eine erfindungsgemäß verwendete Detektoreinrichtung dargestellt. Die Detektoreinrichtung weist vier Einzeldetektoren 311, 312, 313 und 314 auf, die in einer zur Retina R konjugierten Ebene angeordnet und mit dem an der Retina R reflektierten Nachweislicht N beaufschlagt sind.

Ausdrücklich wird darauf hingewiesen, daß es aufgrund des zum Bildaufbau verwendeten "Scan-Verfahrens", bei dem das in den gesamten auswertbaren Raumwinkel gestreute bzw. reflektierte Licht erfaßt und zeitsequentiell zum Bildaufbau verwendet wird, nicht erforderlich ist, daß die Detektoren tatsächlich körperlich in einer zur Retina konjugierten Ebene angeordnet sind. Es genügt vielmehr, wenn in dieser Ebene (bzw. bei Fig.4 in einer zur Pupille konjugierten Ebene) bildfeldbestimmende Blenden angeordnet sind, und das durch diese Blenden hindurchtretende Licht mittels eine Zwischenabbildung vermittelnden Elementen, beispielsweise einer Relaisoptik oder Lichtleiter zu den beabstandet angeordneten Detektoren geleitet wird. Insoweit ist, wenn im folgenden von Detektoren die Rede ist, immer auch gemeint, daß statt dessen bildfeldbestimmende Blenden entsprechend ausgebildet sein können.

Gemäß Fig. 2 sind ferner Strahlteiler 301, 302 und 303 vorgesehen. Während vor dem Detektor 311 kein den Strahlengang beeinflussendes Element angeordnet ist, sind vor dem Detektor 312 ein 0°-Analysator 322, vor dem Detektor 313 ein 45°-Analysator 323 und vor dem Detektor 314 ein $\lambda/4$-Plättchen 324 angeordnet.

Bezeichnet man die Ausgangssignale der Detektoren 311 bis 314 mit A-D, so kann man mittels einer nicht dargestellten Synchronisier- und Auswerteeinheit zur Analyse des Polarisationszustandes des reflektierten Lichts die Stokes-Parameter $S_i$ bilden:

$$S_o = A, \quad S_1 = B-A, \quad S_2 = C-A, \quad S_4 = D-A$$

Hieraus kann man zur Beschreibung der Polarisationscharakteristik folgende Größen ableiten:

$$\text{Polarisationsgrad} \qquad P = \sqrt{(S_1{}^2 + S_2{}^2 + S_3{}^2)} \; / \; S_o$$

$$\text{Polarisationsrichtung} \quad \tan 2\Phi = S_2/S_1$$

$$\text{Elliptizität} \qquad \sin 2\tau = S_3/\sqrt{(S_1{}^2 + S_2{}^2 + S_3{}^2)}$$

Die Ermittlung der Polarisationscharakteristik erlaubt die Ermittlung lokaler Defekte und die Hervorhebung anisotroper, z.B. gerichteter Strukturen der Retina, wie etwa die Nervenfaserschicht.

Bei der in Fig. 2 dargestellten Detektoranordnung (bzw. Blendenanordnung) handelt es sich selbstverständlich lediglich um ein Beispiel für eine erfindungsgemäß verwendete Detektoranordnung mit mehreren Detektoren, die in einer Ebene angeordnet sind, die zu dem abzubildenden Objekt, in dem vorliegenden Ausführungsbeispiel der Retina konjugiert ist.

Fig. 3 zeigt ein weiteres Beispiel für eine Detektoranordnung (bzw. eine Anordnung bildfeldbestimmender Blenden) mit Detektoren 41 bis 45 in einer zur Retinaebene konjugierten Ebene. Der Detektor 41 ist ein sog. Hellfeld-Detektor, während die Detektoren 42 bis 45 Dunkelfelddetektoren sind. Bezeichnet man die Ausgangssignale der Detektoren 41 bis 45 mit 41' bis 45', so gibt die Größe

$$(41' + 43')-(44' + 45')$$

die Richtungscharakteristik der Reflexion an.

Selbstverständlich ist es auch möglich, die Detektoren bzw. die bildfeldbestimmenden Blenden nicht in zur Objektebene R konjugierten Ebenen anzuordnen, sondern beispielsweise in zur Pupille P konjugierten Ebenen. In den Fig. 4a bis 4c sind entsprechende Beispiele dargestellt.

Fig. 4a zeigt eine drei Detektoren 41 bis 43 aufweisende Detektoranordnung. Das Ausgangssignal des Detektors 41 ist proportional zum spekularen Anteil, während die Ausgangssignale 42'und 43' der Detektoren 42 und 43 die Streuung unter größeren Winkeln wiedergeben.

Fig. 4b zeigt eine Detektoranordnung mit fünf Einzeldetektoren 41 bis 45, die bei einem konfokalen Aufbau in einer zur Pupillenebene P konjugierten Ebene angeordnet sind. Das Ausgangssignal 41' des Detektors 41 gibt wiederum den spekularen Anteil an, während die Ausgangssignale

$$(42'-43')$$ den diff. Phasenkontrast

und die Ausgangssignale

$$(42'+43')-(44'+45')$$ die Streucharakteristik

wiedergeben.

Fig. 4c zeigt eine ebenfalls in einer zur Pupille P konjugierten Ebene angeordnete Detektoranordnung mit vier kreissektorförmigen Einzeldetektoren 41 bis 44. Die Verknüpfung

$$(41' + 43')-(42' + 44')$$

der Ausgangssignale dieser Detektoren gibt beispielsweise die Links-Rechts-Asymmetrie des Streulichts wieder. Nochmals soll darauf hingewiesen werden, daß vorstehend Detektoranordnung und Anordnung bildfeldbestimmender Blenden als Synonyme verwendet worden sind.

Vorstehend ist die Erfindung anhand von Ausführungsbeispielen ohne Beschränkung des allgemeinen Erfindungsgedankens - durch besondere Arten der Beleuchtung und/oder der Analyse des rückgestreuten Lichts nach den verschiedensten Kriterien wesentlich weitergehende Informationen über das abzubildende Objekt, beispielsweise den Augenhintergrund zu erhalten als dies mit irgendeiner anderen der bekannten Vorrichtungen zur Bilderzeugung möglich ist - beschrieben worden

Innerhalb dieses allgemeinen Erfindungsgedankens sind selbstverständlich die verschiedensten Modifikationen möglich:

Die erfindungsgemäße optische Bildvorverarbeitung kann zusätzlich auch durch speziell gestaltete austauschbare und/oder variable Blenden, wie LCD-Blenden vor den Detektoren, also beispielsweise anstelle oder zusätzlich zu den Elementen 322 bis 324 verfeinert werden.

Die erfindungsgemäße Vorrichtung kann auch ohne "Double-Scanning" arbeiten, insbesondere bei Detektoranordnungen in der Pupillenebene, bei denen auf einen konfokalen Nachweis verzichtet werden kann.

Auch sind erfindungsgemäße Vorrichtungen mit nur einem Detektor, aber entsprechend ausgebildeten Blenden vorstellbar. Auch können bildfeldbestimmende Blenden, die in bestimmten Ebenen austauschbar angeordnet sind, mit feststehenden, in anderen Ebenen angeordneten Detektoren über Lichtleitmittel verbunden sein.

Die Einspiegelung eines positionierbaren zusätzlichen Strahls und/oder der Umfeldbeleuchtung kann auch in anderer Weise als vorstehend beschrieben erfolgen. Auch können andere Pupillenteilungen oder streng konfokale Anordnungen realisiert werden.

Die vorstehende Beschreibung der Erfindung erlaubt eine Realisierung der erfindungsgemäß verwendeten Auswerte- und Synchronisiereinheit, die die einzelnen Signale verknüpft und/oder speichert auch ohne bis ins einzelne gehende Beschreibung eines Ausführungsbeispiels beispielsweise mittels eines Mikrocomputers.

Es versteht sich von selbst, daß die vorstehende Beschreibung eines Laser-Scanning-Ophthalmoskops die erfindungsgemäßen Grundgedanken nicht auf Ophthalmoskop-Anwendungen beschränkt, obwohl sie gerade bei der Beobachtung des Augenhintergrundes wegen der spezifischen, aus der Augenpupille resultierenden Schwierigkeiten besonders vorteilhaft sind. Die erfindungsgemäßen Grundgedanken sind selbstverständlich auch in Geräten einsetzbar, die zur Beobachtung der Cornea dienen, die als Laser-Scanning-Kameras oder als Laser-Scanning-Mikroskope für medizinische und technische Anwendungen ausgebildet sind, sowie in anderen Laser-Scanning-Bildgebern.

**Patentansprüche**

1. Vorrichtung zur Erzeugung von Bildern eines Objekts und insbesondere zur Beobachtung der hinteren Augenabschnitte,

mit einer Beleuchtungs-Einrichtung, deren Licht auf das abzubildende Objekt fokussiert ist, und die vorzugsweise wenigstens einen Laser (21,22) aufweist,

einer Abtasteinrichtung (1,4), die eine Abtastbewegung des Lichts der Beleuchtungs-Einrichtung auf dem abzubildenden Objekt erzeugt,

einer Detektoreinrichtung (311-314; 41-45) mit wenigstens einem Detektor, die das an dem abzubildenden Objekt reflektierte Licht empfängt, und

einer Auswerte- und Synchronisiereinheit, die aus dem zeitsequentiellen Ausgangssignal der Detektoreinrichtung das Bild erzeugt,

dadurch **gekennzeichnet**, daß die Detektoreinrichtung mehrere Einzeldetektoren (311-314; 41-45) aufweist, die zur Erfassung des von unterschiedlichen Ebenen reflektierten Lichts und/oder zur Erfassung der flächenhaften Intensitätsverteilung des Lichts in einer Ebene und/oder zur Erfassung des Polarisationszustandes des Lichts konjugiert zu unterschiedlichen Ebenen bzw. zu unterschiedlichen Bereichen einer Ebene angeordnet bzw. denen entsprechende Blenden oder Polarisationsfilter vorgeschaltet sind.

2. Vorrichtung nach Anspruch 1,

dadurch **gekennzeichnet**, daß die Beleuchtungs-Einrichtung (21,22) Licht mehrerer Wellenlängen gleichzeitig auf das abzubildende Objekt (R) projiziert, und

daß für das Licht jeder Wellenlänge wenigstens ein und insbesondere ein wellenlängenselektiver Einzeldetektor vorgesehen ist.

3. Vorrichtung nach Anspruch 2,

dadurch **gekennzeichnet**, daß die Eintritts- und/oder Austrittspupillen (51,52) der Lichtstrahlen unterschiedlicher Wellenlänge unterschiedlich sind.

4. Vorrichtung nach Anspruch 3,

dadurch **gekennzeichnet**, daß die Abbildungen mit Licht unterschiedlicher Wellenlänge unterschiedliche Schärfentiefen aufweisen.

5. Vorrichtung nach einem der Ansprüche 1 bis 4,

dadurch **gekennzeichnet**, daß die Auswerteeinheit die Ausgangssignale der Einzeldetektoren auf einem oder mehreren Monitoren darstellt.

6. Vorrichtung nach einem der Ansprüche 1 bis 5,

dadurch **gekennzeichnet**, daß die Auswerteinheit die Ausgangssignale der Einzeldetektoren in Echtzeit verknüpft, und das verknüpfte Signal auf einem Monitor darstellt.

7. Vorrichtung nach einem der Ansprüche 1 bis 5,

dadurch **gekennzeichnet**, daß die Auswerteeinheit die Ausgangssignale der Einzeldetektoren in Bildspeicher einliest und die gespeicherten Signale verknüpft.

8. Vorrichtung nach einem der Ansprüche 1 bis 7,

dadurch **gekennzeichnet**, daß die Detektoreinrichtung das an dem abzubildenden Objekt reflektierte Licht über die Abtasteinrichtung (1,5) sowie gegebenenfalls vorgesehene Blendeneinrichtungen empfängt.

9. Vorrichtung nach einem der Ansprüche 1 bis 8,

dadurch **gekennzeichnet**, daß bei einer Beobachtung des Augenhintergrunds in einer zur Pupille (P) des Auges konjugierten Ebene eine Detektoranordnung vorgesehen ist, die die Intensitätsverteilung des reflektierten Lichts in dieser Ebene erfaßt.

10. Vorrichtung nach Anspruch 9,

dadurch **gekennzeichnet**, daß die Auswerteeinheit aus den Ausgangssignalen der Einzeldetektoren charakteristische Merkmale der Intensitätsverteilung, wie etwa den Schwerpunkt des reflektierten Lichts, Richtungsasymmetrien usw bestimmt.

11. Vorrichtung nach Anspruch 10,

dadurch **gekennzeichnet**, daß die Einzeldetektoren kreissektorförmig ausgebildet und in Form eines Kreises angeordnet sind.

12. Vorrichtung nach einem der Ansprüche 1 bis 11,

dadurch **gekennzeichnet**, daß in einer zum abzubildenden Objekt konjugierten Ebene eine Detektoranordnung vorgesehen ist, die die Intensitätsverteilung des reflektierten Lichts in dieser Ebene erfaßt.

13. Vorrichtung nach Anspruch 12,

dadurch **gekennzeichnet**, daß die Auswerteinheit aus den Ausgangssignalen der Einzeldetektoren charakteristische Merkmale der Intensitätsverteilung, wie etwa den Anteil der Querstreuung innerhalb der Retina oder deren Richtungsasymmetrie ermittelt.

14. Vorrichtung nach einem der Ansprüche 1 bis 13,
dadurch **gekennzeichnet**, daß in einer vorzugsweise zum abzubildenden Objekt (R) konjugierten Ebene eine Reihe von Einzeldetektoren (311..314) vorgesehen sind, denen zumindest zum Teil Polarisatoren (322,323) und/ oder optisch wirksame planparallele Platten (324) vorgeschaltet sind.

15. Vorrichtung nach Anspruch 14,
dadurch **gekennzeichnet**, daß vier Einzeldetektoren vorgesehen sind,
daß dem ersten Einzeldetektor kein optisch wirksames Element, dem zweiten Einzeldetektor ein erster linearer Analysator, dem dritten Einzeldetektor ein zweiter linearer Analysator, der zu dem ersten Analysator um 45° gedreht ist, und dem vierten Einzeldetektor ein /4-Plättchen vorgeschaltet sind, und
daß die Auswerteinheit aus den Ausgangssignalen der vier Detektoren den Polarisationsgrad, die Polarisationsrichtung und die Elliptizität ermittelt.

16. Vorrichtung nach einem der Ansprüche 1 bis 15,
dadurch **gekennzeichnet**, daß der Detektoreinrichtung wenigstens teilweise strukturierte Filter und/oder variable Blenden vorgeschaltet sind.

17. Vorrichtung nach Anspruch 16,
dadurch **gekennzeichnet**, daß elektronisch ansteuerbare flächenhafte Lichtmodulatoren die Filter bilden.

18. Vorrichtung nach Anspruch 17,
dadurch **gekennzeichnet**, daß die Lichtmodulatoren LCD-Elemente mit getrennt ansteuerbaren Bereichen sind.

19. Vorrichtung nach einem der Ansprüche 16 bis 18,
dadurch **gekennzeichnet**, daß die Filter längs und/oder quer zum Nachweis-Strahlengang verschiebbar oder drehbar sind.

20. Vorrichtung nach einem der Ansprüche 1 bis 19,
dadurch **gekennzeichnet**, daß zusätzlich Marken auf das zu abzubildende Objekt und insbesondere den Augenhintergrund projizierbar sind.

21. Vorrichtung nach Anspruch 20,
dadurch **gekennzeichnet**, daß die Projektion von Marken durch Modulation des Beleuchtungslichts erfolgt.

22. Vorrichtung nach Anspruch 21,
dadurch **gekennzeichnet**, daß zur Projektion der Marken eine weitere Lichtquelle und/oder eine weitere Ablenkeinheit bzw. Lichtstrahl-Positioniereinheit (12) vorgesehen ist.

23. Vorrichtung nach Anspruch 22,
dadurch **gekennzeichnet**, daß die Ablenkeinheit einen akusto-optischen Demodulator oder eine Taumeleinheit aufweist.

24. Vorrichtung nach einem der Ansprüche 20 bis 23,
dadurch **gekennzeichnet**, daß die Projektion von Marken zur Fundusperimetrie erfolgt.

25. Vorrichtung nach einem der Ansprüche 1 bis 24,
dadurch **gekennzeichnet**, daß die Abtasteinrichtung eine x/y-Abtasteinrichtung ist, die in an sich bekannter Weise eine drehbare Polygonspiegeltrommel (1) und einen Galvanometerspiegel (5) aufweist.

26. Vorrichtung nach einem der Ansprüche 1 bis 25,
dadurch **gekennzeichnet**, daß zur Umfeldbeleuchtung eine weitere Lichtquelle vorgesehen ist, deren Licht (B) das abzubildende Objekt und insbesondere den Augenhintergrund großflächig beleuchtet.

27. Vorrichtung nach einem der Ansprüche 1 bis 26,
dadurch **gekennzeichnet**, daß der Strahl eines Bearbeitungslasers und insbesondere eines Koagulationslasers zusätzlich eingespiegelt ist.

28. Vorrichtung nach einem der Ansprüche 1 bis 27,
dadurch **gekennzeichnet**, daß eine Steuereinheit den Beobachtungsstrahlengang und/oder den Strahl des Bearbeitungslasers bei Bewegungen des abzubildenden Objekts und insbesondere bei Augenbewegungen nachführt (Eye-Tracking) oder den Koagulationslaser bei Augenbewegungen während der Koagulation abschaltet.

29. Vorrichtung nach Anspruch 28,
dadurch **gekennzeichnet**, daß die Steuereinheit zusätzlich eine Einstellung der Bearbeitungsparameter und insbesondere eine Behandlungsplanung ermöglicht.

30. Vorrichtung nach einem der Ansprüche 1 bis 29,
dadurch **gekennzeichnet**, daß der Detektoreinrichtung eine Bildfeldblende vorgeschaltet ist, die wenigstens für einen Teil der Einzeldetektoren eine Dunkelfeldbeleuchtung erzeugt.

31. Vorrichtung nach einem der Ansprüche 1 bis 30,
dadurch **gekennzeichnet**, daß in das aufgenommene Bild ein früher aufgenommenes Bild des abzubildenden Objekts und insbesondere eine Fundus-Angiogramm und/oder Markierungen deckungsgleich einblendbar sind.

32. Vorrichtung nach einem der Ansprüche 1 bis 31,
dadurch **gekennzeichnet**, daß die Steuereinheit eine Bildverarbeitungseinrichtung für die Verarbeitung der verschiedenen gleichzeitig und nacheinander aufgenommenen Bilder aufweist.

33. Vorrichtung nach einem der Ansprüche 1 bis 32,
dadurch **gekennzeichnet**, daß die Beleuchtungs-Einrichtung, die Abtasteinrichtung und die Detektoreinrichtung eine konfokale Anordnung bilden, und
daß die Bildfeldblende wenigstens einiger Einzeldetektoren wesentlich größer als der Punktbilddurchmesser auf dem Augenhintergrund ist.

34. Vorrichtung nach einem der Ansprüche 1 bis 33,
dadurch **gekennzeichnet**, daß in den zu den unterschiedlichen Ebenen konjugierten Ebenen bildfeldbestimmende Blenden und die Detektoren davon beabstandet ortsfest angeordnet sind.

35. Vorrichtung nach Anspruch 34,
dadurch **gekennzeichnet**, daß Lichtleitmittel die Blenden und die Detektoren verbinden.

## Claims

1. A device to generate images of an object and in particular to observe the fundus of the eye,
with an illumination device whose light is focused on the object to be imaged and which preferably possesses at least one laser (21,22),
with a scanning device (1,4) which generates a scanning motion of the light of the illumination device on the object to be imaged,
with a detecting device (311-314; 41-45) with at least one detector which receives the light reflected at the object to be imaged, and
with an evaluation and synchronization unit which generates the image from the temporally sequential output signal of the detecting device,
characterized by the fact that the detecting device possesses several single detectors (311-314; 41-45) which are positioned in a plane or which have corresponding diaphragms or polarizing filters positioned before them so that they can track the light reflected from the different planes and/or track the areal intensity distribution of the light in one plane and/or track the polarizing status of the light conjugated to different planes or to different areas of one plane.

2. A device in accordance with claim 1,
characterized by the fact that the illumination device (21,22) simultaneously projects light of several wavelengths onto the object to be imaged (R), and that at least one, and in particular one wavelength-selective single detector is provided for the light of each wavelength.

3. A device in accordance with claim 2,
characterized by the fact that the entrance and/or exit pupils (51,52) of the light beams of different wavelengths are different.

4. A device in accordance with claim 3,
characterized by the fact that with light of different wavelengths the images show different depth of focus.

5. A device according to any of claims 1 to 4,
characterized by the fact that the evaluation unit displays the output signals of the single detectors on one or more monitors.

6. A device according to any of claims 1 to 5,
characterized by the fact that the evaluation unit links up the output signals of the single detectors in real time and displays the linked signal on a monitor.

7. A device according to any of claims 1 to 5,
characterized by the fact that the evaluation unit reads the output signals of the single detectors into the image store and links the stored signals.

8. A device according to any of claims 1 to 7,
characterized by the fact that the detecting device receives the light reflected on the object to be imaged via the scanning device (1,5) and via the diaphragm devices provided as the case may be.

9. A device according to any of claims 1 to 8,
characterized by the fact that for an observation of the fundus of the eye in a plane conjugated to the pupil (P) of the eye a detector array is provided which tracks the intensity distribution of the reflected light in this plane.

10. A device in accordance with claim 9,
characterized by the fact that the evaluation unit determines characteristic features of the intensity distribution such as the point of concentration of the reflected light, directional asymmetries, etc. from the output signals of the single detectors.

11. A device in accordance with claim 10,
characterized by the fact that the single detectors are designed in circle sector shape and are positioned in the form of a circle.

12. A device according to any of claims 1 to 11,
characterized by the fact that in a place conjugated to the object to be imaged a detector array is provided which tracks the intensity distribution of the reflected light in this plane.

13. A device in accordance with claim 12,
characterized by the fact that the evaluation unit determines characteristic features of the intensity distribution such as the share of the lateral scattering within the retina or its directional asymmetry from the output signals of the single detectors.

14. A device according to any of claims 1 to 13,
characterized by the fact that in a plane preferably conjugated to the object to be imaged (R) a series of single detectors (311..314) is provided in front of which at least in part polarizers (322,323) and/or optically effective planoparallel plates (324) are positioned.

15. A device in accordance with claim 14,
characterized by the fact that four single detectors are provided,
that no optically effective element is positioned before the first single detector, a first linear analyzer is positioned in front of the second single detector, a second linear analyzer is positioned in front of the third single detector which analyzer is rotated through 45° over the first analyzer, and a λ/4 plate is positioned in front of the fourth single detector, and
that the evaluation unit deter-nines the polarization direction and the ellipticity from the output signals of the four detectors.

16. A device according to any of claims 1 to 15,
characterized by the fact that at least some structured filters and/or variable diaphragms are positioned in front of the detecting device.

17. A device in accordance with claim 16,
characterized by the fact that electronically controllable areal light modulators form the alters.

18. A device in accordance with claim 17,
characterized by the fact that the light modulators are LCD elements with separately controllable bands.

19. A device according to any of claims 16 to 18,
characterized by the fact that the alters can be moved or turned lengthways and/or laterally to the reference beam path.

20. A device according to any of claims 1 to 19,
characterized by the fact that in addition marks can be projected onto the object to be imaged and particularly onto the fundus.

21. A device in accordance with claim 20,
characterized by the fact that the projection of marks is performed by the modulation of the illuminating light.

22. A device in accordance with claim 21,
characterized by the fact that a further light source and/or a further deflection unit or light beam positioning unit (12) is provided to project the marks.

23. A device in accordance with claim 22,
characterized by the fact that the deflection unit possesses an acousto-optical demodulator or a wobble unit.

24. A device according to any of claims 20 to 23,
characterized by the fact that the projection of marks is made to the fundus perimeter.

25. A device according to any of claims 1 to 24,
characterized by the fact that the scanning unit is an x/y scanning unit which possesses in a known fashion a rotatable polygon mirror drum (1) and a galvanometer mirror (5).

26. A device according to any of claims 1 to 25,
characterized by the fact that for background illumination a further light source is provided whose light (B) illuminates the object to be imaged and in particular the fundus over a wide area.

27. A device according to any of claims 1 to 26,
characterized by the fact that the beam of a treatment laser and in particular of a coagulation laser is additionally imaged.

28. A device according to any of claims 1 to 27,
characterized by the fact that a control unit tracks the observation beam path and/or the beam of the treatment laser on movements of the object to be imaged and in particular on eye movements (eye-tracking) or switches

EP 0 290 566 B1

off the coagulation laser on eye movements during coagulation.

29. A device in accordance with claim 28,
characterized by the fact that the control unit additionally makes possible a setting of the treatment parameters and in particular a treatment plan.

30. A device according to any of claims 1 to 29,
characterized by the fact that the detecting device has an image field diaphragm situated in front of it which generates a dark field illumination for at least a part of the single detectors.

31. A device according to any of claims 1 to 30,
characterized by the fact that a picture of the object to be imaged taken earlier and in particular a fundus angiogram and/or markings can be mixed into the picture taken in a flush position.

32. A device according to any of claims 1 to 31,
characterized by the fact that the control unit possesses an image processing unit for the processing of the different pictures taken simultaneously and sequentially.

33. A device according to any of claims 1 to 32,
characterized by the fact that the illumination device, the scanning unit and the detecting unit for a confocal array, and
that the image field diaphragm of at least some single detectors is substantially larger than the point image diameter on the fundus.

34. A device according to any of claims 1 to 33,
characterized by the fact that image field determining diaphragms and the detectors positioned at a distance to these are positioned at a fixed position in the planed conjugated to the different planes.

35. A device in accordance with claim 34,
characterized by the fact that light-conducting agents connect the diaphragms and the detectors.


**Revendications**

1. Dispositif destiné à produire des images d'un objet, et en particulier à l'observation des parties arrière de l'oeil, comportant
un dispositif d'éclairage,dont la lumière est focalisée sur l'objet dont il s'agit de former l'image et qui présente de préférence au moins un laser (21, 22),
un dispositif d'exploration (1, 4),qui génère un déplacement exploratoire de la lumière du dispositif d'éclairage sur l'objet dont il s'agit de former l'image,
un dispositif de détection (311 à 314 ; 41 à 45) comportant au moins un détecteur qui reçoit la lumière réfléchie sur l'objet dont il s'agit de former l'image, et
une unité d'exploitation et de synchronisation, qui produit l'image à partir du signal de sortie, séquentiel dans le temps, émis par le dispositif de détection,
caractérisé en ce que le dispositif de détection présente plusieurs détecteurs individuels (311 à 314 ; 41 à 45) qui sont disposés en vue de capter la lumière réfléchie par des plans différents et/ou de capter la répartition superficielle de l'intensité de la lumière dans un plan et/ou de capter l'état de polarisation de la lumière, de façon à être conjugués avec des plans différents, ou avec des zones différentes d'un plan ou en amont desquels sont montés des diaphragmes ou des filtres de polarisation correspondants.

2. Dispositif selon la revendication 1,
caractérisé en ce que le dispositif d'éclairage (21, 22) projette simultanément, sur l'objet (R) dont il s'agit de former l'image, de la lumière de plusieurs longueurs d'onde et en ce que, pour la lumière de chaque longueur d'onde, on prévoit au moins un détecteur individuel et en particulier un détecteur individuel opérant la sélection de longueur d'onde.

3. Dispositif selon la revendication 2,
caractérisé en ce que les pupilles d'entrée et/ou de sortie (51, 52) des rayons lumineux de longueurs d'onde différentes sont différentes.

4. Dispositif selon la revendication 3,
caractérisé en ce que les images formées avec de la lumière de longueurs d'onde différentes présentent des profondeurs de champ différentes.

5. Dispositif selon une des revendications 1 à 4,
caractérisé en ce que l'unité d'exploitation présente, sur un ou plusieurs moniteurs, les signaux de sortie des détecteurs individuels.

6. Dispositif selon une des revendications 1 à 5,
caractérisé en ce que l'unité d'exploitation combine en temps réel les signaux de sortie des détecteurs individuels et présente sur un moniteur le signal combiné.

7. Dispositif selon une des revendications 1 à 5,
caractérisé en ce que l'unité d'exploitation enregistre les signaux de sortie des détecteurs individuels dans des mémoires d'images et combine les signaux enregistrés.

8. Dispositif selon une des revendications 1 à 7
caractérisé en ce que le dispositif de détection reçoit, par l'intermédiaire du dispositif d'exploration (1, 5) ainsi que par l'intermédiaire des dispositifs de diaphragmes éventuellement prévus, la lumière réfléchie sur l'objet dont il s'agit de former l'image.

9. Dispositif selon une des revendications 1 à 8,
caractérisé en ce que, dans le cas d'une observation du fond de l'oeil dans un plan conjugué avec la pupille (P) de l'oeil, on prévoit un dispositif de détection qui capte la répartition, dans ce plan de l'intensité de la lumière réfléchie.

10. Dispositif selon la revendication 9,
caractérisé en ce que l'unité d'exploitation détermine, à partir des signaux de sortie des détecteurs individuels, les caractéristiques de la répartition de l'intensité, comme par exemple le centre de gravité de la lumière réfléchie, des asymmétries selon l'orientation, etc.

11. Dispositif selon la revendication 10,
caractérisé en ce que les détecteurs individuels ont la forme de secteurs circulaires et sont disposés en formant un cercle.

12. Dispositif selon une des revendications 1 à 11,
caractérisé en ce que, dans un plan conjugué à l'objet dont il s'agit de former l'image, on prévoit un dispositif de détection,qui capte la répartition dans ce plan de l'intensité de la lumière réfléchie.

13. Dispositif selon la revendication 12,
caractérisé en ce que l'unité d'exploitation établit, à partir des signaux de sortie des détecteurs individuels, quelles sont certaines caractéristiques de la répartition de l'intensité, comme par exemple la proportion de la dispersion transversale à l'intérieur de la rétine, ou son asymétrie selon l'orientation.

14. Dispositif selon une des revendications 1 à 13,
caractérisé en ce que, dans un plan conjugué de préférence avec l'objet (R) dont il s'agit de former l'image, on prévoit une série de détecteurs individuels (311 à 314) en amont desquels sont montés des polariseurs (322, 323) au moins partiels et/ou des plaques planes parallèles (324) optiquement actives.

15. Dispositif selon la revendication 14,
caractérisé en ce que l'on prévoit quatre détecteurs individuels,
en ce qu'en amont du premier détecteur individuel, on ne monte pas d'élément actif optiquement,
en ce qu'en amont du deuxième détecteur individuel on monte un premier analyseur linéaire, en amont du troisième détecteur individuel on monte un deuxième analyseur linéaire, qui est tourné de 45° par rapport au premier analyseur individuel et en ce qu'en amont du quatrième détecteur individuel on monte une plaquette λ/4 (quart d'onde)
et en ce que l'unité d'exploitation établit, à partir des signaux de sortie des quatre détecteurs, quel est le degré de polarisation, quelle est la direction de polarisation et quelle est l'ellipticité.

16. Dispositif selon l'une des revendications 1 à 15,
caractérisé en ce que des filtres au moins partiellement structurés et/ou des diaphragmes variables sont montés en amont du dispositif de détection.

17. Dispositif selon la revendication 16,
caractérisé en ce que des modulateurs superficiels de lumière, susceptibles d'être commandés électroniquement forment les filtres.

18. Dispositif selon la revendication 17,
caractérisé en ce que les éléments de modulation de la lumière sont des éléments LCD (diodes à cristaux liquides), comportant des zones susceptibles d'être commandées séparément.

19. Dispositif selon une des revendications 16 à 18,
caractérisé en ce que les filtres peuvent être déplacés en translation longitudinalement et/ou transversalement par rapport au chemin optique de détection, ou ils peuvent tourner.

20. Dispositif selon une des revendications 1 à 19,
caractérisé en ce que des repères supplémentaires peuvent être projetés sur l'objet dont il s'agit de former l'image, et en particulier sur le fond de l'oeil.

21. Dispositif selon la revendication 20,
caractérisé en ce que la projection des repères est effectuée en modulant la lumière d'éclairage.

22. Dispositif selon la revendication 21,
caractérisé en ce qu'en vue de la projection des repères, on prévoit une autre source de lumière et/ou une

**14**

autre unité de déviation ou unité (12) de positionnement du rayon lumineux.

23. Dispositif selon la revendication 22,
caractérisé en ce que l'unité de déviation présente un démodulateur acousto-optique ou une unité de nutation.

24. Dispositif selon une des revendications 20 à 23,
caractérisé en ce que la projection de repères est effectuée en vue de la périmétrie du fond de l'oeil.

25. Dispositif selon une des revendications 1 à 24,
caractérisé en ce que le dispositif d'exploration est un dispositif d'exploration x/y qui présente, (de façon connue en elle-même), un tambour (1) à miroir polygonal tournant, et un miroir (5) de galvanométrie.

26. Dispositif selon une des revendications 1 à 25,
caractérisé en ce qu'en vue de l'éclairage du champ environnant, on prévoit une autre source de lumière, dont la lumière (B) éclaire sur une grande surface l'objet dont il s'agit de former l'image, et en particulier le fond de l'oeil.

27. Dispositif selon une des revendications 1 à 26,
caractérisé en ce que le rayon d'un laser de traitement, et en particulier d'un laser de coagulation est envoyé en plus.

28. Dispositif selon une des revendications 1 à 27,
caractérisé en ce qu'une unité de commande guide en réaction, dans le cas de mouvements de l'oeil ("eye-tracking") le trajet du rayon d'observation et/ou le rayon du laser de traitement, ou coupe le laser de coagulation dans le cas de mouvements de l'oeil au cours de la coagulation.

29. Dispositif selon la revendication 28,
caractérisé en ce que l'unité de commande permet de plus le réglage des paramètres de traitement, et en particulier la programmation du traitement.

30. Dispositif selon une des revendications 1 à 29,
caractérisé en ce qu'en amont du dispositif de détection est monté un diaphragme de champ d'image, qui produit un éclairage de champ sombre au moins pour une partie des détecteurs individuels.

31. Dispositif selon une des revendications 1 à 30,
caractérisé en ce que dans l'image reçue, on peut mélanger une image, reçue précédemment, de l'objet dont il s'agit de former l'image et en particulier un angiogramme du fond de l'oeil et/ou des repères, et ce, avec un recouvrement parfait.

32. Dispositif selon une des revendications 1 à 31,
caractérisé en ce que l'unité de commande présente un dispositif de traitement d'image/destiné au traitement des différentes images reçues simultanément et successivement.

33. Dispositif selon une des revendications 1 à 32,
caractérisé en ce que le dispositif d'éclairage, le dispositif d'exploration et le dispositif de détection forment un dispositif cofocal, et en ce que le diaphragme de champ d'image d'au moins quelques détecteurs individuels est sensiblement plus grand que le diamètre d'image ponctuelle sur le fond de l'oeil.

34. Dispositif selon une des revendications 1 à 33,
caractérisé en ce que, dans les plans conjugués avec les différents plans, on met en place de façon fixe des diaphragmes déterminant le champ d'image, et en ce que les détecteurs sont situés à une certaine distance de ceux-ci.

35. Dispositif selon la revendication 34,
caractérisé en ce que des moyens à conducteurs optiques (ou formés par des conducteurs optiques) relient les diaphragmes aux détecteurs.

Fig. 1

*Fig. 2*

EP 0 290 566 B1

Fig.3

Fig.4a

Fig.4b

Fig.4c

Fig.5

18